# EUROPÄISCHE PATENTSCHRIFT

(11) **EP 2 091 320 B1**
(45) Veröffentlichungstag und Bekanntmachung des Hinweises auf die Patenterteilung: **26.10.2011**
(21) Anmeldenummer: 07819776.1
(22) Anmeldetag: 13.11.2007
(51) Int. Cl.: A01K 1/015, A01N 65/00, A01N 25/08, A61K 31/473, A01N 43/00, C05F 17/00

(54) **SORPTIONSMEDIUM Verfharen zur Herstellung und Anwendung**
SORPTION MEDIUM process of production und use
AGENT DE SORPTION procédé de production et utilisation

(30) Priorität: 13.11.2006 AT 18762006
(43) Veröffentlichungstag der Anmeldung: 26.08.2009
(73) Patentinhaber: Neufeld, Klaus, 2532 Heiligenkreuz (AT); Grabherr, Helmut, 4550 Kremsmünster (AT); Westerkamp, Arnold, 49429 Visbek (DE)
(72) Erfinder: NEUFELD, Klaus, 2532 Heiligenkreuz (AT)
(74) Vertreter: Puchberger, Peter
(86) Internationale Anmeldenummer: PCT/EP2007/009790
(87) Internationale Veröffentlichungsnummer: WO 2008/058697

(56) Entgegenhaltungen:
- JP-A- 2001 095 410
- JP-A- 2006 297 284
- US-A- 5 044 324

## Beschreibung

Die Erfindung bezieht sich auf spezielle Sorptionsmedien und deren Einsatz und Verwendung.

In letzter Zeit haben Einstreumaterialien in der landwirtschaftlichen Nutztierproduktion zunehmend an Bedeutung gewonnen. Der Trend zu einer tierartgerechten Tierhaltung führte dazu, dass vermehrt Tiere in Gruppen- oder in Bodenhaltung gehalten werden. Auch gesetzliche Richtlinien, die den Aspekt des Tierschutzes verfolgen, haben vermehrt dazu geführt, dass die Notwendigkeit zur Verwendung geeigneter und insbesondere auch tierschutzgerechter Einstreumaterialien Bedeutung gewinnt.

Bisher wurden in der Nutztierhaltung keine oder kostengünstige Einstreumaterialien verwendet, die lokal verfügbar waren, wie etwa Stroh, Sägespäne, Sägemehl oder Hobelspäne. In früheren Zeiten war auch die Verwendung von Laub durchaus gängig, was allerdings später aufgrund der arbeitsintensiven Beschaffung kaum mehr zur Anwendung kam. Diese Materialien wurden und werden ebenso im Bereich der Haltung von Hobbytieren, insbesondere Pferden, eingesetzt. Bei der Haltung von Kleintieren, wie etwa Kaninchen, Meerschweinchen etc., werden vor allem Einstreumaterialien aus Hobelspänen, Stroh, Maisspindel etc. eingesetzt. Abweichend davon werden bei der Haltung von Hauskatzen für die Befüllung der sogenannten Katzenklos vor allem flüssigkeitsbindende Materialien auf Basis von Tonmineralen und anderen mineralischen Substanzen eingesetzt, es kommen aber auch Produkte aus Holz, Stroh, Maisspindeln und anderen organischen Materialien zur Anwendung.

Die Nachteile der herkömmlich verwendeten Einstreumaterialien sind vor allem darin gelegen, dass sie keine ausreichende oder nachhaltige Flüssigkeitsbindungskapazität haben und dass von den Tieren ausgeschiedene Stickstoffverbindungen (z.B. Harnstoff, Harnsäure) mikrobiell fermentiert und teilweise zu flüchtigen Schadgasen (z.B. Ammoniak, Schwefelverbindungen) umgebildet werden. Dieser Fermentationsprozess ist umso höher, je höher der Wassergehalt und die Temperatur des Mediums sind. Die entstehenden Schadgase gelten als Prädispositionsfaktoren für viele Erkrankungen (insbesondere des Respirationstraktes) und andere Probleme bei den betroffenen Tieren, wie etwa lokale Hautreizungen und Vorschädigungen der Haut, die das Eindringen von Mikroorganismen (Bakterien, Viren, Pilze) erleichtern. Bei Huf- und Klauentieren können diese volatilen Noxen auch zu einer Schädigung des Huf- bzw. Klauenhorns führen und somit Ursprung von Erkrankungen des Bewegungsapparats sein, die in vielen Fällen letal enden oder zumindest eine schwerwiegende Leistungsdepression der Tiere herbeiführen.

Weiters führen die Fermentationsprozesse im Einstreumaterial zu einer raschen Vermehrung der Mikroorganismen und damit zu einer starken mikrobiellen Belastung, wobei Bakterien, Pilze und deren Toxine in weiterer Folge über die Stailluft in Aerosolform die Gesundheit der Tiere und des Betreuungspersonals belasten. Die entstehenden Schadgase (Ammoniak, Schwefelverbindungen) sowie Mikroorganismen und Toxine, welche die Stailluft beeinträchtigen, müssen durch Belüftungssysteme auf eine erträgliche Konzentration reduziert werden. Im Winter muss die frisch zugeführte Außenluft erwärmt werden. All dies führt zu einem erheblichen Energieverbrauch.

Die im Geflügelbereich hauptsächlich eingesetzte Stalleinstreu sind Hobelspäne. Diese müssen in einer relativ dicken Schicht eingestreut werden, um aufgrund ihrer schlechten physikalischen Eigenschaften einen einigermaßen wirksamen Effekt zu erzielen. Das führt in der kalten Jahreszeit dazu, dass die Isolationswirkung der Einstreuschicht zu einer Verlagerung des Taupunktes im Bereich des Stallbodens in das Einstreumaterial führt, was zu einer weiteren Durchfeuchtung der Einstreu durch Kondensatbildung führt.

Außerdem haben viele der üblichen Einstreumaterialien aufgrund ihrer Struktur und physikalischen Eigenschaften sowie aufgrund der Tatsache, dass sie schon *per se* mikrobiell kontaminiert sind, weitere erhebliche Nachteile. Dies sind etwa die Schadwirkung von scharfkantigen Hobelspänen auf die sehr sensible Ballenhaut von jungem Geflügel, die unzureichende Flüssigkeitsbindungskapazität von Hobelspänen und Stroh und die oft hochgradige Kontamination von Stroh mit Pilzen und Pilztoxinen. Aufgrund dieser unzureichenden Eigenschaften und der negativen Auswirkung bei manchen Tieren, z.B. Truthahn, besteht das Bestreben aufseiten des Gesetzgebers, aus Gründen des Tierschutzes in gewissen Produktionsbereichen die Tierzahl pro Flächeneinheit zu reduzieren. Dies würde in der Folge für den Landwirt zu einer wesentlichen Verteuerung des Produktionsprozesses und für den Konsumenten zu einer Verteuerung der landwirtschaftlichen Produkte führen.

Weiters ist bei all diesen Materialien ein sehr hoher Arbeitsaufwand nötig, da durch die ungünstigen physikalischen Eigenschaften häufig nachgestreut werden muss, was für die Tiere eine Beunruhigung und für den Landwirt einen zusätzlichen Arbeitsaufwand darstellt. In weiterer Folge entstehen sehr große Mengen von Stalleinstreu, die teilweise schwer kompostierbar ist und somit ein erhebliches ökonomisches Problem bei der Entsorgung darstellt.

Es wurden verschiedene Einstreumaterialien entwickelt, die die genannten Nachteile mehr oder weniger kompensieren sollten. So ist in US 6 435 135 ein Einstreumaterial für Geflügel aus komprimierten Holzfasern beschrieben. In US 6 837 181 ist eine Tiereinstreu aus Holzpartikeln aus Pinien- und Pappelholz genannt. In US 5 271 355 wird die Verwendung von Pappelholz in Kombination mit Torf genannt In US 5 542 374 wird eine Kombination von Ton und Zedernholz beschrieben. In US 5 884 584 ist eine Tiereinstreu aus einer Kombination von Pinienholz, Luzerne und Pappelholz genannt.

Aus technischer Sicht ist die Verwendung verschiedener Zusatzstoffe möglich, die einerseits die Ureaseaktivität hemmen oder andererseits die mikrobielle Aktivität im Einstreumedium reduzieren. Die meisten bekannten Mittel sind jedoch chemischer Natur und können daher in der Regel im Bereich der Nutztierproduktion nicht eingesetzt werden. Es ist daher von besonderer Bedeutung, dass alle Komponenten und Wirkstoffe, die im Einstreubereich für Nutztiere verwendet werden, futtermittelrechtlich unbedenklich sind.

Die Erfindung schlägt ein verbessertes, alkaloidhaltiges Sorptionsmedium vor, das eine lignocellulosehaltige Sorptionskomponente und mindestens ein Isochinolinalkaloid, vorzugsweise mindestens ein Benzophenanthridinalkaloid, enthält.

Die Isochinolinalkaloide können in Form von Pflanzenmaterialien, wie Rhizomen, Blätter, Stängel und dergleichen, enthalten sein. Beispielsweise können für die vorliegende Erfindung Pflanzenmaterialien von Papaveraceae verwendet werden. Als besonders bevorzugte Pflanzen kann man beispielsweise *Sanguinaria canadensis, Macleaya cordata, Chelidonium majus, Hydrastis canadensis* etc. nennen. Die Papaveraceae enthalten Isochinolinalkaloide, insbesondere Benzophenanthridinalkaloide, wie beispielsweise Sanguinarin und Chelerythrin.

Erfindungsgemäß können auch Extrakte aus Pflanzenmaterialien, wie beispielsweise der *Sanguinaria canadensis* oder der *Macleaya cordata* oder aus anderen Papaveraceae, enthalten sein. Gemäß einer bevorzugten Ausführungsform der Erfindung werden Extrakte aus der *Macleaya cordata* eingesetzt.

Erfindungsgemäß geeignete Extrakte aus Pflanzenmaterialien können nach jedem bekannten Extraktionsverfahren erhalten werden, wie beispielsweise wässrige Extraktion, alkoholische Extraktion, CO₂-Extraktion und dergleichen.

Erfindungsgemäß können in dem Sorptionsmedium auch Salze oder Derivate isolierter Isochinolinalkaloide oder ihre synthetischen Analoga enthalten sein.

Das erfindungsgemäße Sorptionsmedium enthält vorzugsweise Sanguinarin und Chelerythrin, wobei die Sanguinarin-Dosierung von 0,001 ppm bis 1500 ppm, bevorzugt 0,02 bis 250 ppm beträgt.

Das Verhältnis Sanguinarin zu Chelerythrin in für die vorliegende Erfindung bevorzugt verwendeten Extrakten kann beispielsweise von 1:100 bis 100:1 variieren. Ein Verhältnis von ca. zwei Teilen Sanguinarin zu ca. einem Teil Chelerythrin ist eine besonders geeignete Zusammensetzung.

Gemäß einem weiteren Merkmal der Erfindung kann vorgesehen sein, dass die Sorptionskomponente des Sorptionsmediums aus lignocellulosehaltigen Materialien ausgewählt ist. Beispiele für solche lignocellulosehaltigen Materialien sind Holz- und Rindenpartikel und deren Mischungen. Aber auch andere Materialien, wie etwa Stroh, Schilf oder andere lignocellulosehaltige Pflanzenmaterialien können erfindungsgemäß in der Sorptionskomponente eingesetzt werden.

Es kann erfindungsgemäß vorgesehen sein, dass das lignocellulosehaltige Material in unverarbeiteter Form oder in verarbeiteter Form eingesetzt wird.

Wenn die Sorptionskomponente des erfindungsgemäßen Sorptionsmediums vermahlene Holz- oder Rindenpartikel enthält, kann die Partikelgröße beispielsweise kleiner als 10 mm, bevorzugt kleiner als 1,6 mm sein. Die verwendeten Hölzer können alle Arten von Hölzern sein. Bevorzugt werden Hölzer von heimischen Koniferenarten, wie beispielsweise Fichte, Kiefer oder Lärche, verwendet. Als Rinden können alle Rinden sowohl von Weich- als auch von Harthölzern eingesetzt werden. Bevorzugt wird jedoch die Rinde von Kiefernarten für die Sorptionskomponente verwendet.

Die vermahlene Sorptionskomponente, die ein lignocellulosehaltiges Material ist, wird mit isochinolinalkaloidhaltigem Pflanzenmaterial oder Extrakten aus diesem, insbesondere Pflanzenmaterial der *Sanguinaria canadensis* oder der *Macleaya cordata* oder Extrakten daraus, in der angegebenen Dosierung der Isochinolinalkaloide vermischt, anschließend kompaktiert, beispielsweise pelletiert, und in der Folge gekrümelt, d.h. wieder auf eine gewünschte Partikelgröße gebrochen. Diese Partikelgröße kann beispielsweise zwischen 0,001 und 35 mm, bevorzugt zwischen 0,05 und 10 mm betragen. Idealerweise werden staubförmige Anteile des Materials ausgesiebt.

Der isochinolinalkaloidhaitige Extrakt kann in gelöster Form auf die Sorptionskomponente aufgesprüht werden. Dies kann vor oder auch nach der Verpressung erfolgen. Der Extrakt kann auch auf das fertige, d.h. auf die gewünschte Partikelgröße gebrochene Produkt aufgesprüht werden.

Die vorliegende Erfindung betrifft auch ein Verfahren zur Herstellung des erfindungsgemäßen Sorptionsmediums. Das Verfahren beinhaltet folgende Schritte:
(a) Reinigen und Trocknen der Sorptionskomponente,
(b) Zerkleinern der Sorptionskomponente, gegebenenfalls Aussiebung auf eine gewünschte Korngröße,
(c) Herstellen eines Zwischenproduktes durch Vermischen der in Schritt (b) erhaltenen Sorptionskomponente mit isochinolinalkaloidhaltigem Pflanzenmaterial oder durch Aufsprühen eines isochinolinalkaloidhaltigen Extraktes auf die im Schritt (b) erhaltene Sorptionskomponente,
(d) Kompaktieren des im Schritt (c) erhaltenen Zwischenproduktes und anschließend Krümeln auf die gewünschte Partikelgröße.

Gemäß einem weiteren Merkmal der Erfindung kann vorgesehen sein, dass das Verfahren folgende Schritte beihaltet:
(a) Reinigen und Trocknen der Sorptionskomponente,
(b) Zerkleinern der Sorptionskomponente, gegebenenfalls Aussiebung auf eine gewünschte Korngröße,
(c) Kompaktieren des im Schritt (b) erhaltenen Produktes und anschließend Krümeln auf die gewünschte Partikelgröße,
(d) Aufsprühen eines isochinolinalkaloidhaltigen Extrakts auf die Sorptionskomponente.

Die vorliegende Erfindung hat auch eine Einstreu für einen Käfig, einen Einstellplatz, eine Stallbox etc. für ein Nutz-, Heim- oder Hobbytier zum Gegenstand, wobei die Einstreu ein alkaloidhaltiges Sorptionsmedium, wie es oben definiert wurde, enthält.

Gemäß einem weiteren Merkmal der Erfindung kann vorgesehen sein, dass eine solche Einstreu das/die Isochinolinalkaloid/e in wirksamer Menge zur Heilung, Linderung oder Prophylaxe von Erkrankungen der Ballenhaut, der Hufe und Klauen von Nutz-, Heim- oder Hobbytieren enthält. Die wirksame Menge kann in einem weiten Bereich variieren. Die Sanguinarin-Dosierung kann beispielsweise im Bereich 0,001 bis 1500 ppm liegen.

Die vorliegende Erfindung hat auch ein Geruchs- und Flüssigkeitsbindemittel für Bioabfallsammelbehälter zum Gegenstand, das ein erfindungsgemäßes alkaloidhaltiges Sorptionsmedium enthält. Vorzugsweise enthält das Geruchs- und Flüssigkeitsbindemittel für Bioabfallsammelbehälter das/die Isochinolinalkaloidie in wirksamer Menge zur Reduktion oder Vermeidung von mikrobiellem Wachstum in dem Bioabfallsammelbehälter. Beispielsweise kann das alkaloidhaltige Sorptionsmedium Sanguinarin in einer Menge von 0,001 bis 1500 ppm enthalten.

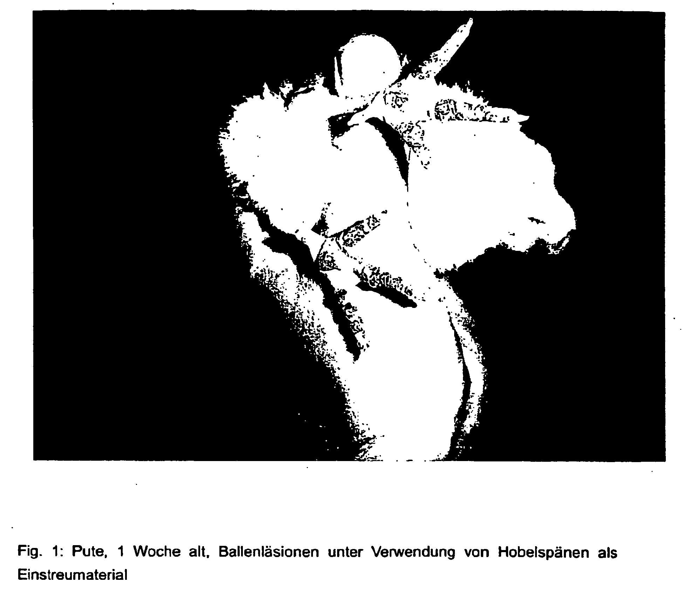

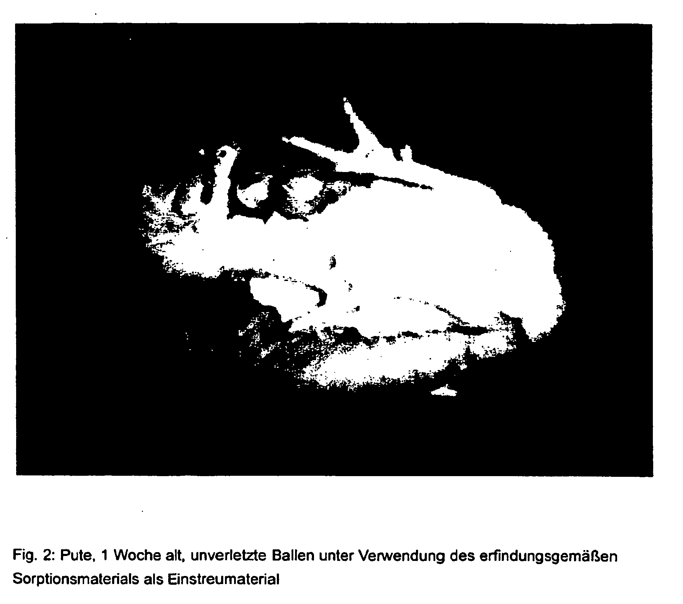

Die Erfindung wird im Folgenden unter Bezugnahme auf spezielle Ausführungsbeispiele, Beispiele und die Figuren erläutert, wobei Fig. 1 die Ballenläsionen einer 1 Woche alten Pute bei Verwendung eines herkömmlichen Einstreumaterials und Fig. 2 die unverletzten Ballen einer 1 Woche alten Pute bei Verwendung des erfindungsgemäßen Sorptionsmediums als Einstreumaterial zeigt.

Bevorzugte Ausführungsbeispiele des erfindungsgemäßen Sorptionsmediums sind wie folgt:
Sorptionsmedium 1: Die Sorptionskomponente ist Kiefernrinde und die Isochinolinalkaloide sind in Form eines Extrakts aus *Macleaya cordata* enthalten.
Sorptionsmedium 2: Die Sorptionskomponente ist eine Mischung aus Kiefernrinde und Holz, vorzugsweise Fichten- und/oder Kiefernholz, und die Isochinolinalkaloide sind in Form eines Extrakts aus *Macleaya cordata* enthalten.
Sorptionsmedium 3: Die Sorptionskomponente ist eine Mischung aus Fichten- und Kiefemholz und die Isochinolinalkaloide sind in Form eines Extrakts aus *Macleaya cordata* enthalten.
Sorptionsmedium 4: Die Sorptionskomponente ist vermahlenes Kiefemholz und die Isochinolinalkaloide sind in Form eines Extrakts aus *Macleaya cordata* enthalten.
Sorptionsmedium 5: Die Sorptionskomponente ist vermahlenes Fichtenholz und die Isochinolinalkaloide sind in Form eines Extrakts aus *Macleaya cordata* enthalten.
Sorptionsmedium 6: Die Sorptionskomponente ist vermahlenes Lärchenholz und die Isochinolinalkaloide sind in Form eines Extrakts aus *Macleaya cordata* enthalten.
Sorptionsmediums 7: Die Sorptionskomponente ist eine Mischung aus Lärchenrinde und Kiefernrinde und die Isochinolinalkaloide sind in Form eines Extrakts aus *Macleaya cordata* enthalten,

Die Partikelgrößenverteilung bei den genannten Sorptionsmedien 1 bis 7 können beispielsweise wie folgt sein: 0% der Partikel > 8,0 mm, 78-83% der Partikel > 3,15 mm, 15-18% der Partikel > 2.0 mm, 0-1% der Partikel < 2,0 mm.

Partikel dieser Art weisen beispielsweise einen Feuchtigkeitsgehalt von 8,50-9,30% auf und haben ein Feuchtigkeitsbindungsvermögen von ca. dem 5- oder einem höheren Mehrfachen ihres Eigengewichtes.

### Bespiel 1: Herstellung eines erfindungsgemäßen Sorptionsmediums

Die Sorptionskomponente, d.h. Holz oder Rinde oder eine Mischung davon, wird gereinigt, von Fremdanteilen befreit, anschließend auf einen Trockensubstanzgehalt von etwa 10% getrocknet und zerkleinert. Gegebenenfalls kann eine Absiebung auf eine gewünschte Korngröße erfolgen. Anschließend wird der isochinolinalkaloidhaltige Extrakt aufgesprüht oder das isochinolinalkaloidhaltige Pflanzenmaterial beigemischt. Nach der Beimischung der alkaloidhaltigen Substanz wird das Material kompaktiert und danach in einem Krümler auf eine gewünschte Partikelgröße gekrümelt.

Alternativ dazu kann das gereinigte, zerkleinere, getrocknete und gegebenenfalls auf eine gewünschte Korngröße ausgesiebte Sorptionsmaterial kompaktiert und anschließend gekrümelt werden und eine lösliche Form des Extraktes dann in einem letzten Schritt auf die fertige Sorptionskomponente aufgesprüht werden.

Gemäß einer Ausgestaltung der Erfindung eignet sich dieses alkaloidhaltige Sorptionsmedium beispielsweise als Einstreumaterial für Einstellplätze, Käfige, Stallboxen etc. von Nutztieren, Hobbytieren oder Heimtieren. Es erfüllt die Forderung, dass alle Komponenten und Wirkstoffe, die im Einstreubereich für Tiere, insbesondere Nutztiere, verwendet werden, futtermittelrechtlich unbedenklich sein müssen. Die Wirksamkeit des erfindungsgemäßen Sorptionsmediums, insbesondere hinsichtlich Produktstabilität und sonstiger technischer Eigenschaften, konnte bisher mit keiner anderen futtermittelrechtlich unbedenklichen Stoffkombination erreicht werden. Weiters hat das erfindungsgemäße Sorptionsmedium den Vorteil, dass es bei voller Wirksamkeit nur in dünner Schicht auf den Stallboden aufgebracht werden muss und es somit nicht, wie etwa bei Hobelspänen, zu einer Taupunktverlagerung aus dem Stallboden in die Einstreuschicht kommt, was zu einer zusätzlichen Durchfeuchtung des Einstreumaterials aus Hobelspänen führt.

Weiters stellt in der landwirtschaftlichen Nutztierproduktion, aber auch etwa bei Hobbytieren, wie dem Pferd, der Arbeitsaufwand zur Entmistung und zum Einstreuen der Ställe einen erheblichen Kostenfaktor dar. Bei der erfindungsgemäßen Einstreu ist es möglich, erhebliche Kosten im Hinblick auf den Arbeitsaufwand einzusparen, da das arbeitsintensive Ausmisten und Neueinstreuen vermieden werden kann.

Die antimikrobiellen Eigenschaften vieler Isochinolinalkaloide sind bekannt, insbesondere die antimikrobiellen Eigenschaften von Sanguinarin. In dem erfindungsgemäßen Sorptionsmedium, das eine lignocellulosehaltige Sorptionskomponente und Isochinolinalkaloide, insbesondere Sanguinarin und Chelerythrin, enthält, kommt es zu überraschenden Effekten, welche die Wirksamkeit des Einstreumediums um ein Vielfaches verbessern. Schon in Dosierungen, bei denen es noch zu keiner nachweisbaren antimikrobiellen Wirkung kommt, ist die Aktivität der Urease im erfindungsgemäßen Einstreumaterial deutlich reduziert. Dadurch wird auch die Bildung von schädlichem Ammoniak deutlich reduziert. Dies hat zur Folge, dass einerseits das Einstreumaterial länger verwendet werden kann, andererseits die oben erwähnten, durch Ammoniak bedingten Probleme deutlich reduziert sind.

Dies sind vor allem Erkrankungen des Respirationstraktes bei allen Tierarten, Läsionen der Haut und Hautanhangsgebilde der Gliedmaßen von Geflügel, Schädigungen von Huf- und Klauenhorn von Pferden und anderen Huftieren bzw. Klauentieren. Diese Schädigungen führen zu erheblichen Gesundheitsstörungen und Leistungseinbußen bei landwirtschaftlichen Nutztieren.

Als Endprodukt des Stickstoffstoffwechsels werden bei Tieren Harnstoff oder Harnsäure mit dem Urin ausgeschieden. Dieser Harnstoff wird durch mikrobielle Aktivität mittels von den Bakterien gebildeter Urease in großer Menge zu Ammoniak umgebildet, das gasförmig ist. D.h. die Konzentration von Harnstoff in der Einstreu nimmt durch diesen Prozess dramatisch ab. Harnstoff ist jedoch granulationsfördernd und hat bei Gewebeverletzungen einen positiven Einfluss auf die Wundheilung. So wird Harnstoff auch bei verschiedenen Hauterkrankungen wie Psoriasis, Neurodermitis aber auch in Kosmetika als Feuchthaltefaktor für die Hornhaut eingesetzt. Wird nun in der Stalleinstreu vermehrt Harnstoff in Ammoniak umgebildet, kommt es nicht nur durch das Ammoniak zu einer schädigenden Wirkung auf das Gewebe direkt, sondern es kommt auch zu einer Reduktion des Harnstoffes und damit zu einer Verminderung dessen protektiver Eigenschaften auf die Hornhaut. Besonders krass ist dieser Effekt bei Geflügel. Geflügel scheidet in den ersten sechs Lebenswochen wie Säugetiere Harnstoff aus. Die Ballenhaut von jungem Geflügel ist sehr empfindlich und wird durch das Fehlen von Harnstoff, durch ungeeignetes Einstreumaterial (scharfkantig) und durch die reizende Wirkung von Ammoniak leicht beeinträchtigt. Es kommt daher bei Geflügel frühzeitig zu Ballenläsionen, die in der Folge wiederum das Eindringen von Keimen fördern. Dies führt zu schmerzhaften Gelenkserkrankungen der Extremitäten, wodurch Bewegungsstörungen und Fressunlust bedingt werden. Aber die negativen Effekte von ungeeignetem Einstreumaterial sind nicht nur aus tierschützerischer Sicht problematisch, die beschriebenen Faktoren führen in weiterer Folge auch zu Leistungseinbußen. Dieses Problem tritt besonders deutlich bei Puten auf, da diese durch die lange Mastdauer und das hohe Mastendgewicht durch Schmerzen im Bewegungsapparat stark beeinträchtigt werden. Durch die Verwendung eines erfindungsgemäßen Sorptionsmediums als Einstreumaterial kann dieses Problem entschärft werden.

### Beispiel 2: Feldversuch zur Ermittlung des Einflusses von Einstreumaterial auf die Entstehung von Pododermatitis bei Mastputen

In einem Versuch mit 170 Mastputen wurde der Einfluss des Einstreumaterials auf die Tiergesundheit, insbesondere auf das Entstehen von Ballenverletzungen überprüft. Dazu wurde ein ca. 20 m² großes Stallabteil mit herkömmlicher Einstreu (Hobelspänen) bis zu einer Höhe von 3 cm eingestreut (Kontrollgruppe), in einem zweiten, ebenso großen Abteil wurde ein erfindungsgemäßes Sorptionsmedium mit Kiefernrinde als Sorptionskomponente und mit einem Gehalt von 1,5 g Sanguinarin pro Tonne Sorptionsmedium als Einstreu in gleicher Weise eingebracht (Versuchsgruppe).

In jedem Abteil wurden 85 Küken untergebracht. In beiden Abteilen wurde die Einstreu bei Durchnässung bei Bedarf entfernt und durch frische Einstreu ersetzt. Futter, Fütterungsmanagement, Stallklima und Lichtprogramm entsprachen den üblichen Rahmenbedingungen in einem Putenmastbetrieb. Erhoben wurden Futter- und Wasseraufnahme der Tiere, Leistungsparameter, Ausfälle, Gesundheitsstatus insbesondere Anzeichen von Pododermatitis, Einstreuverbrauch und Stallklimadaten.

Der Versuch ergab, dass die Versuchsgruppe signifikant geringeres Auftreten von Ballenverletzungen, insbesondere Anzeichen von Pododermatitis, verzeichnete als die Kontrollgruppe.

Die Fig. 1 zeigt die Ballenläsionen bei einer 1 Woche alten Pute, die aus der Verwendung eines herkömmlichen Einstreumaterials, nämlich der Verwendung von Hobelspänen, folgen (Kontrollgruppe). Die Fig. 2 zeigt die unverletzten Ballen einer 1 Woche alten Pute, die bei Verwendung eines erfindungsgemäßen Sorptionsmediums als Einstreumaterial zu beobachten sind.

Gemäß einer weiteren Ausgestaltung der Erfindung eignet sich das alkaloidhaltige Sorptionsmedium auch als Geruchs- und Feuchtigkeitsbindemittel in Behältern zum Sammeln von Bioabfällen.

Bei der Sammlung von verrottbaren und kompostierbaren Abfällen (Biomüll), wie Küchenabfällen, kommt es zu einem Absetzen von Flüssigkeit aus den Abfällen. Das. Fehlen von trockenem, schwer verrottbarem Abfallmaterial und fehlender Durchlüftung führt zu starkem mikrobiellen Wachstum, insbesondere Wachstum von gesundheitsschädlichen Pilzen. Diese Keime, insbesondere die Pilze und deren Toxine, belasten in der Folge die Raumluft. Dies ist meistens die Luft der Küche. Durch die beschriebene mikrobielle Belastung und durch die Zersetzung der Abfälle entstehen schlechte Gerüche, die besonders bei heißer Witterung unangenehm auftreten oder aber im Bereich der Küche störend sind.

Durch ein das erfindungsgemäße Sorptionsmedium enthaltendes Geruchs- und Flüssigkeitsbindemittel in Bioabfallbehältern und Biomülltonnen wird eine Flüssigkeitsansammlung in diesen Behältnissen vermieden und mikrobielles Wachstum in diesen Behältnissen und die damit einhergehender Belastung der Luft vermieden oder reduziert. Außerdem kommt es durch das Hinanhalten von mikrobiellem Wachstum zu einer Reduktion oder Vermeidung von schlechten Gerüchen.

In einem erfindungsgemäßen Geruchs- und Flüssigkeitsbindemittel kann Sanguinarin beispielsweise in einer Menge von 0,001 bis 1500 ppm enthalten sein.

## Patentansprüche

1. Alkaloidhaltiges Sorptionsmedium, **dadurch gekennzeichnet, dass** es eine lignocellulosehaltige Sorptionskomponente und mindestens ein Isochinolinalkaloid, vorzugsweise mindestens ein Benzophenanthridinalkaloid, enthält.

2. Alkaloidhaltiges Sorptionsmedium nach Anspruch 1, **dadurch gekennzeichnet, dass** die Isochinolinalkaloide in Form von Pflanzenmaterialien, wie Rhizomen, Blätter, Stängel, oder als Extrakte aus solchen Pflanzenmaterialien oder als Salze oder Derivate isolierter Isochinolinalkalolde oder in Form der synthetischen Analoga enthalten sind.

3. Alkaloidhaltiges Sorptionsmedium nach Anspruch 1 oder 2, **dadurch gekennzeichnet, dass** die Isochinolinalkaloide in Form von Pflanzenmaterialien und/oder in Form von Extrakten aus Pflanzenmaterial von Papaveraceae, vorzugsweise ausgewählt aus der aus *Sanguinaria canadensis, Macleaya cordata, Chelidonium majus, Hydrastis canadensis* und deren Mischungen bestehenden Gruppe, enthalten ist.

4. Alkaloidhaltiges Sorptionsmedium nach einem der Ansprüche 1 bis 3, **dadurch gekennzeichnet, dass** Sanguinarin und Chelerythrin enthalten sind.

5. Alkaloidhaltiges Sorptionsmedium nach einem der Ansprüche 1 bis 4, **dadurch gekennzeichnet, dass** Sanguinarin in einer Menge von 0,001 bis 1500 ppm, vorzugsweise 0,02 bis 250 ppm, enthalten ist.

6. Alkaloidhaltiges Sorptionsmedium nach einem der Ansprüche 1 bis 5, **dadurch gekennzeichnet, dass** die Sorptionskomponente unverarbeitetes oder verarbeitetes lignocellulosehaltiges Material enthält.

7. Alkaloidhaltiges Sorptionsmedium nach einem der Ansprüche 1 bis 6, **dadurch gekennzeichnet, dass** das lignocellulosehaltige Material vorzugsweise aus der aus Weichhölzern, Harthölzern, Rinden von Weichhölzern, Rinden von Harthölzern und deren Mischungen bestehenden Gruppe ausgewählt ist.

8. Alkaloidhaltiges Sorptionsmedium nach einem der Ansprüche 1 bis 7, **dadurch gekennzeichnet, dass** die Sorptionskomponente Hölzer von Koniferenarten enthält, vorzugsweise ausgewählt aus der Gruppe bestehend aus Fichtenholz, Kiefemholz, Lärchenholz und deren Mischungen.

9. Alkaloidhaltiges Sorptionsmedium nach einem der Ansprüche 1 bis 7, **dadurch gekennzeichnet, dass** die Sorptionskomponente Rinden von Kiefernarten enthält.

10. Verfahren zur Herstellung eines alkaloidhaltigen Sorptionsmediums gemäß einem der Ansprüche 1 bis 9, **gekennzeichnet durch** folgende Schritte:
(a) Reinigen und Trocknen der Sorptionskomponente,
(b) Zerkleinern der Sorptionskomponente, gegebenenfalls Aussiebung auf eine Größe unter 10 mm, vorzugsweise unter 1,6 mm,
(c) Herstellen eines Zwischenproduktes **durch** Vermischen der in Schritt (b) erhaltenen Sorptionskomponente mit isochinolinalkaloidhaltigem Pflanzenmaterial oder **durch** Aufsprühen eines isochinolinalkaloidhaltigen Extraktes auf die im Schritt (b) erhaltene Sorptionskomponente,
(d) Kompaktieren des im Schritt (c) erhaltenen Zwischenproduktes und anschließend Krümeln auf eine Partikelgröße zwischen 0,001 und 35 mm, bevorzugt zwischen 0,05 und 10 mm.

11. Verfahren zur Herstellung eines alkaloidhaitigen Sorptionsmediums gemäß einem der Ansprüche 1 bis 9, **gekennzeichnet durch** folgende Schritte:
(a) Reinigen und Trocknen der Sorptionskomponente,
(b) Zerkleinern der Sorptionskomponente, gegebenenfalls Aussiebung auf eine Größe unter 10 mm, vorzugsweise unter 1,6 mm,
(c) Kompaktieren des im Schritt (b) erhaltenen Produktes und anschließend Krümeln auf eine Partikelgröße zwischen 0,001 und 35 mm, bevorzugt zwischen 0,05 und 10 mm,
(d) Aufsprühen eines isochinolinalkaloidhaltigen Extrakts auf die Sorptionskomponente.

12. Einstreu für einen Käfig, einen Einstellplatz, eine Stallbox etc. für ein Nutz-, Heimoder Hobbytier, **dadurch gekennzeichnet, dass** sie ein alkaloidhaltiges Sorptionsmedium gemäß einem der Ansprüche 1 bis 9 enthält.

13. Einstreu nach Anspruch 12, **dadurch gekennzeichnet, dass** Sanguinarin in einer Menge von 0,001 bis 1500 ppm enthalten ist.

14. Verwendung eines alkaloidhaltigen Sorptionsmediums nach einem der Ansprüche 1 bis 9 zur Herstellung einer Einstreu zur Heilung, Linderung oder Prophylaxe von Erkrankungen der Ballenhaut, der Hufe und Klauen von Nutz-, Heim- oder Hobbytieren.

15. Geruchs- und Flüssigkeitsbindemittel für Bioabfallsammelbehälter, **dadurch gekennzeichnet, dass** ein alkaloidhaltiges Sorptionsmedium gemäß einem der Ansprüche 1 bis 9 enthalten ist.

16. Geruchs- und Flüssigkeitsbindemittel nach Anspruch 15, **dadurch gekennzeichnet, dass** Sanguinarin in einer Menge von 0,001 bis 1500 ppm enthalten ist.

17. Verwendung eines Geruchs- und Flüssigkeitsbindemittels nach Anspruch 15 oder 16 zur Reduktion oder Vermeidung von mikrobiellem Wachstum in dem Bioabfallbehälter.

## Claims

1. Alkaloid-containing sorption medium, **characterized in that** it contains a lignocellulosic sorption component and at least one isoquinoline alkaloid, preferably at least one benzophenanthridine alkaloid.

2. Alkaloid-containing sorption medium according to Claim 1, **characterized in that** the isoquinoline alkaloids are present in the form of plant materials, such as rhizomes, leaves, stems, or as extracts of such plant materials, or as salts or derivatives of isolated isoquinoline alkaloids, or in the form of the synthetic analogues.

3. Alkaloid-containing sorption medium according to Claim 1 or 2, **characterized in that** the isoquinoline alkaloids are present in the form of plant materials and/or in the form of extracts of plant material from Papaveraceae, preferably selected from the group consisting of *Sanguinaria canadensis, Macleaya cordata, Chelidoniurn majus, Hydrastis canadensis* and mixtures thereof.

4. Alkaloid-containing sorption medium according to any one of Claims 1 to 3, **characterized in that** sanguinarine and chelerythrine are present.

5. Alkaloid-containing sorption medium according to any one of Claims 1 to 4, **characterized in that** sanguinarine is present in an amount of 0.001 to 1500 ppm, preferably 0.02 to 250 ppm.

6. Alkaloid-containing sorption medium according to any one of Claims 1 to 5, **characterized in that** the sorption component contains unprocessed or processed lignocellulosic material.

7. Alkaloid-containing sorption medium according to any one of Claims 1 to 6, **characterized in that** the lignocellulosic material is preferably selected from the group consisting of softwoods, hardwoods, barks of softwoods, barks of hardwoods, and mixtures thereof.

8. Alkaloid-containing sorption medium according to any one of Claims 1 to 7, **characterized in that** the sorption component contains woods of conifer species, preferably selected from the group consisting of spruce, pine, larch and mixtures thereof.

9. Alkaloid-containing sorption medium according to any one of Claims 1 to 7, **characterized in that** the sorption component contains barks of pine species.

10. Method for producing an alkaloid-containing sorption medium according to any one of Claims 1 to 9, **characterized by** the following steps:
(a) cleaning and drying the sorption component,
(b) comminuting the sorption component, optionally sieving to a size below 10 mm, preferably below 1.6 mm,
(c) producing an intermediate by mixing the sorption component obtained in step (b) with isoquinoline alkaloid-containing plant material, or by spraying an isoquinoline alkaloid-containing extract onto the sorption component obtained in step (b),
(d) compacting the intermediate obtained in step (c) and subsequently crumbling it to a particle size between 0.001 and 35 mm, preferably between 0.05 and 10 mm.

11. Method for producing an alkaloid-containing sorption medium according to any one of Claims 1 to 9, **characterized by** the following steps:
(a) cleaning and drying the sorption component,
(b) comminuting the sorption component, optionally sieving to a size below 10 mm, preferably below 1.6 mm,
(c) compacting the product obtained in step (b) and subsequently crumbling it to a particle size between 0.001 and 35 mm, preferably between 0.05 and 10 mm,
(d) spraying an isoquinoline alkaloid-containing extract onto the sorption component.

12. Litter for a cage, a stable, a box stall etc. for a farm animal, pet or hobby animal, **characterized in that** it contains an alkaloid-containing sorption medium according to any one of Claims 1 to 9.

13. Litter according to Claim 12, **characterized in that** sanguinarine is present in an amount of 0.001 to 1500 ppm.

14. Use of an alkaloid-containing sorption medium according to any one of Claims 1 to 9 for producing a litter for therapy, amelioration or prophylaxis of disorders of the foot pad skin, hoof and claws of farm animals, pets or hobby animals.

15. Odour-binding agent and liquid-binding agent for biowaste collecting vessel, **characterized in that** an alkaloid-containing sorption medium according to any one of Claims 1 to 9 is present.

16. Odour-binding agent and liquid-binding agent according to Claim 15, **characterized in that** sanguinarine is present in an amount of 0.001 to 1500 ppm.

17. Use of an odour-binding agent and liquid-binding agent according to Claim 15 or 16 for reducing or avoiding microbial growth in the biowaste vessel.

## Revendications

1. Agent de sorption alcaloïdique, **caractérisé en ce qu'**il contient un constituant de sorption lignocellulosique et au moins un alcaloïde isoquinoléinique, de préférence au moins un alcaloïde benzophénanthridinique.

2. Agent de sorption alcaloïdique suivant la revendication 1, **caractérisé en ce que** les alcaloïdes isoquinoléiniques sont contenus sous la forme de matières végétales, comme des rhizomes, des feuilles, des tiges, ou sous la forme d'extraits de matières végétales de ce genre, ou sous la forme de sels ou de dérivés d'alcaloïdes isoquinoléiniques isolés ou sous la forme d'analogues synthétiques.

3. Agent de sorption alcaloïdique suivant la revendication 1 ou 2, **caractérisé en ce que** les alcaloïdes isoquinoléiniques sont contenus sous la forme de matières végétales et/ou sous la forme d'extraits de matières végétales de papavéracéae, choisies de préférence dans le groupe constitué de *Sanguinarin canadensis, Macleaya cordata, Chelidonium majus, Hydrastis canadensis* et de leurs mélanges.

4. Agent de sorption alcaloïdique suivant l'une des revendications 1 à 3, **caractérisé en ce qu'**il contient de la sanguinarine et de la chélérythrine.

5. Agent de sorption alcaloïdique suivant l'une des revendications 1 à 4, **caractérisé en ce qu'**il contient de la sanguinarine en une quantité de 0,001 à 1500 ppm, de préférence de 0,02 à 250 ppm.

6. Agent de sorption alcaloïdique suivant l'une des revendications 1 à 5, **caractérisé en ce que** l'agent de sorption contient de la matière lignocellulosique non traitée ou traitée.

7. Agent de sorption alcaloïdique suivant l'une des revendications 1 à 6, **caractérisé en ce que** la matière lignocellulosique est choisie, de préférence, dans le groupe constitué de bois tendres, de bois durs, d'écorces de bois tendres, d'écorces de bois durs et de leurs mélanges.

8. Agent de sorption alcaloïdique suivant l'une des revendications 1 à 7, **caractérisé en ce que** le constituant de sorption contient des bois de type conifère, choisis de préférence dans le groupe constitué du bois d'épicéa, du bois de pin, du bois de mélèze et de leur mélanges.

9. Agent de sorption alcaloïdique suivant l'une des revendications 1 à 7, **caractérisé en ce que** le constituant de sorption contient des écorces de type pin.

10. Procédé de préparation d'un agent de sorption alcaloïdique suivant l'une des revendications 1 à 9, **caractérisé par** les stades suivants :
(a) on purifie et on sèche le constituant de sorption,
(b) on fragmente le constituant de sorption, le cas échéant en le tamisant jusqu'à une dimension inférieure à 10 mm, de préférence inférieure à 1,6 mm,
(c) on prépare un produit intermédiaire en mélangeant le constituant de sorption obtenu au stade (b) à de la matière végétale contenant au moins un alcaloïde isoquinoléinique ou en projetant un extrait contenant au moins un alcaloïde isoquinoléinique sur le constituant de sorption obtenu au stade (b),
(d) on compacte le produit intermédiaire obtenu au stade (c) et ensuite on l'émiette jusqu'à une granulométrie comprise entre 0,001 et 35 mm, de préférence comprise entre 0,05 et 10 mm.

11. Procédé de préparation d'un agent de sorption alcaloïdique suivant l'une des revendications 1 à 9, **caractérisé par** les stades suivants :
(a) on purifie et on sèche le constituant de sorption,
(b) on fragmente le constituant de sorption, le cas échéant en le tamisant jusqu'à une dimension inférieure à 10 mm, de préférence inférieure à 1,6 mm,
(c) on compacte le produit intermédiaire obtenu au stade (b) et ensuite on l'émiette jusqu'à une granulométrie comprise entre 0,001 et 35 mm, de préférence comprise entre 0,05 et 10 mm,
(d) on projette un extrait contenant au moins un alcaloïde isoquinoléinique sur le constituant de sorption.

12. Litière pour une cage, une logette, une case etc. pour un animal utile, un animal domestique ou un animal de compagnie, **caractérisée en ce qu'**elle contient un agent de sorption alcaloïdique suivant l'une des revendications 1 à 9.

13. Litière suivant la revendication 12, **caractérisée en ce qu'**elle contient de la sanguinarine en une quantité de 0,001 à 1500 ppm.

14. Utilisation d'un agent de sorption alcaloïdique suivant l'une des revendications 1 à 9, pour la production d'une litière pour la guérison, le soulagement ou la prophylaxie de maladies de l'oignon ou coussinet, du sabot et des griffes ou onglons d'animaux utiles, d'animaux domestiques ou d'animaux de compagnie.

15. Agent de fixation des odeurs et des liquides pour des récipients de collecte de déchets biologiques, **caractérisé en ce qu'**il contient un agent de sorption alcaloïdique suivant l'une des revendications 1 à 9.

16. Agent de fixation des odeurs et des liquides suivant la revendication 15, **caractérisé en ce qu'**il contient de la sanguinarine en une quantité de 0,001 à 1500 ppm.

17. Utilisation d'un agent de fixation des odeurs et des liquides suivant la revendication 15 ou 16, pour réduire ou empêcher la croissance microbienne dans le récipient de déchets biologiques.
